# EUROPEAN PATENT SPECIFICATION

(11) **EP 2 205 159 B1**
(45) Date of publication and mention of the grant of the patent: **13.01.2016**
(21) Application number: 08845439.2
(22) Date of filing: 15.10.2008
(51) Int. Cl.: A61B 8/06, G01S 7/52, G01S 15/89

(54) **METHODS AND APPARATUS FOR ULTRASOUND IMAGING**
ULTRASCHALLABBILDUNGSVERFAHREN UND -VORRICHTUNG
PROCÉDÉS ET APPAREIL D'IMAGERIE PAR ULTRASONS

(30) Priority: 29.10.2007 US 926228
(43) Date of publication of application: 14.07.2010
(73) Proprietor: Hitachi Aloka Medical, Ltd., Mitaka-shi Tokyo 181-8622 (JP)
(72) Inventor: TAMURA, Tadashi, North Haven, Connecticut 06473 (US)
(74) Representative: Heim, Hans-Karl
(86) International application number: PCT/JP2008/069080
(87) International publication number: WO 2009/057486

(56) References cited:
- EP-A2- 1 067 400
- WO-A1-02/47554
- WO-A1-2006/095287
- JP-A- 2001 258 886
- JP-A- 2003 225 238
- JP-A- 2003 319 940
- JP-A- 2007 152 111
- JP-T- 2004 500 915
- US-A1- 2007 164 898
- US-B1- 6 512 854

## Description

### TECHNICAL FIELD

The invention relates generally to the field of ultrasound imaging. More specifically, embodiments of the invention relate to methods and systems for automatically adjusting the gain of Doppler signals used to measure flow velocity.

### BACKGROUND ART

Ultrasound is used to image various organs, heart, liver, fetus, and blood vessels. For diagnosis of cardiovascular diseases, spectral Doppler is usually used to measure blood flow velocity. The pulsed Doppler technique is often used due to its inherent spatial sampling capability which permits the sampling of velocity in a blood vessel compared with continuous-wave (CW) Doppler which does not have spatial discrimination capability and samples all signals along the ultrasound beam. CW Doppler is used especially when a high blood velocity is expected to be measured since CW Doppler is not limited by the pulse repetition frequency (PRF) limits (Nyquist sampling theorem). CW Doppler may still be limited in maximum velocity due to signal sampling when performing analyses such as FFT (fast Fourier transform) and others.

A Doppler system typically transmits ultrasound and detects blood flow velocity as the shift in frequency (Doppler shift frequency) in the received ultrasound signal. The received ultrasound is demodulated with the reference signals as a complex signal having in-phase (I) and quadrature (Q) at the same frequency as the transmitted frequency. After low-pass filtering, high frequency components such as the second harmonics are blocked, passing only baseband signals. Wall-filtering (i.e., high-pass filtering) is applied to the baseband signals to remove clutter noise manifest from stationary tissue and slowly moving tissues such as blood vessel walls, resulting in complex Doppler I-Q signals. The complex I-Q Doppler signals are input to a spectrum analyzer such as an FFT analyzer to obtain the Doppler frequency spectrum which represents blood velocities. Typically, 128-point, 256-point, and 512-point FFTs are used.

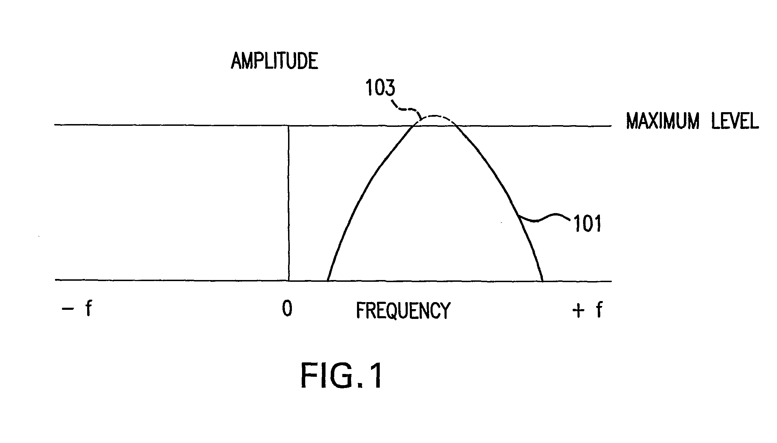

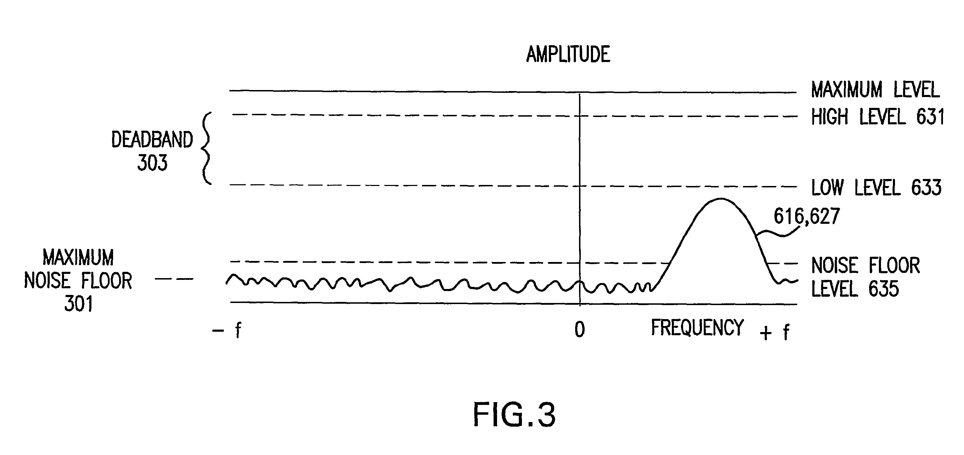

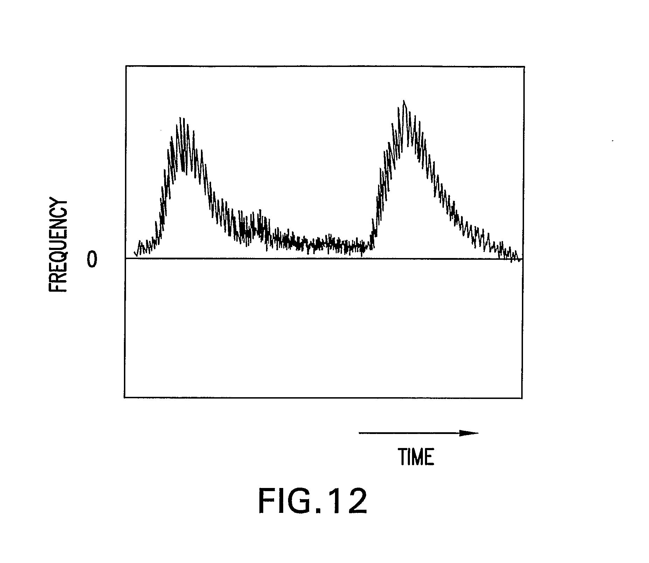

The Doppler spectrum is generally displayed with time as shown in FIG. 12 because of the time varying nature of blood flow. The horizontal axis is time and the vertical axis is frequency. Spectrum power is displayed as the brightness as shown in FIG. 12. The spectrum power can be plotted as the spectrum power vs. frequency at a given time as shown in FIG. 3. The Doppler spectrum may exhibit noise due in part by the ultrasound system electronics and other sources. FIG. 3 shows a Doppler spectrum having a noise floor which is indicative of random noise broadly distributed by an FFT. The noise may mask the true blood flow signal if the Doppler flow signal gain is too low. Conversely, FIG. 1 shows a Doppler spectrum having a Doppler flow signal gain that is too high where the peak Doppler spectrum is clipped.

The Doppler flow signal gain determines the amplitude of the Doppler signal input to an FFT spectrum analyzer. The output of the Doppler spectrum is usually compressed in dynamic range as 8-bit, 12-bit, 16-bit or other resolutions. It can be seen that a proper Doppler flow signal gain output to an ultrasound system improves the Doppler spectrum's SNR (signal-to-noise ratio), thereby improving the image quality when displayed.

Most ultrasound systems today allow a user to manually adjust Doppler gain settings to obtain the best spectrum. However, in adjusting these settings, the user consumes time that could be better spent performing diagnosis. There exists a need to overcome these problems.

US 2007/0164898 A1 discloses a method and an apparatus for automatically adjusting a parameter used in the display of the Doppler spectral image comprising acquiring a plurality of spectral lines of Doppler data.

### SUMMARY OF THE INVENTION

The inventor has discovered that it would be desirable to have a system and method that examines the Doppler spectrum signals output by an ultrasound system when measuring blood flow velocity to determine the proper Doppler gain. Noise present in the Doppler spectrum is examined and used as a criterion for optimal gain. If the Doppler gain is too high or too low according to predetermined levels, the overall gain is adjusted.

One aspect of the invention provides methods for automatically controlling the gain from a Doppler signal processor during ultrasound imaging as specified in claim 1.

Methods according to this aspect of the invention comprise inputting returned ultrasound signals, demodulating the returned ultrasound signals, wall-filtering the returned ultrasound signals producing Doppler flow signals, performing spectral analysis on the Doppler flow signals producing a Doppler spectrum, setting a high level signal threshold, setting a low level signal threshold, setting a noise floor level threshold, detecting a peak Doppler spectrum level and a Doppler spectrum maximum noise floor from the Doppler flow signals, increasing Doppler flow signal gain if the peak Doppler spectrum amplitude is less than the low level signal threshold until the peak Doppler spectrum amplitude equals the high level signal threshold or the maximum noise floor is equal to the noise floor level threshold, and decreasing the Doppler flow signal gain if the peak Doppler spectrum amplitude is greater than the high level signal threshold until the peak Doppler spectrum amplitude equals the high level signal threshold or the maximum noise floor is equal to the noise floor level threshold.

Another aspect of the invention provides systems for automatically controlling the gain of a Doppler spectrum processor during ultrasound imaging as specified in claim 6.

Systems according to this aspect of the invention comprise a receiver configured to receive returned ultrasound signals and having an output, a Doppler signal processor having an input coupled to the receiver output and an output, the Doppler signal processor configured to demodulate and wall-filter the returned ultrasound signals and output Doppler flow signals, a variable gain amplifier having an input coupled to the Doppler signal processor output, a gain control signal input and an output, the variable gain amplifier configured to vary the gain of the Doppler flow signals, a spectrum analyzer having an input coupled to the variable gain amplifier output and an output, the spectrum analyzer configured to convert the Doppler flow signals into their corresponding frequency spectrum, and an automatic gain engine coupled to the spectrum analyzer output, the automatic gain engine configured to receive the Doppler spectrum and detect a peak Doppler spectrum amplitude and a maximum noise floor wherein a gain control signal is calculated and coupled to the variable gain amplifier gain control signal input based on the maximum noise floor present in the Doppler flow signals spectrum and predetermined high, low and noise floor signal level thresholds wherein if the peak Doppler spectrum amplitude is greater than the high level signal threshold, or less than the low level signal threshold, overall gain is adjusted to maintain the peak Doppler spectrum amplitude greater than the low level signal threshold and less than the high level signal threshold.

The details of one or more embodiments of the invention are set forth in the accompanying drawings and the description below. Other features, objects, and advantages of the invention will be apparent from the description and drawings, and from the claims.

### BRIEF DESCRIPTION OF THE DRAWINGS

FIG. 1 is an exemplary high gain Doppler spectrum plot.
FIG. 2 is an exemplary low gain Doppler spectrum plot.
FIG. 3 is an exemplary Doppler spectrum with noise floor.
FIG. 4 is an exemplary noise suppression gain function *g*(*p*).
FIG. 5A is an exemplary Doppler spectrum before noise suppression.
FIG. 5B is an exemplary Doppler spectrum after noise suppression.
FIG. 6 is an exemplary Doppler spectrum processor with the automatic Doppler gain control system and the noise suppressor.
FIG. 7 is an exemplary flow chart to describe the automatic Doppler gain control method.
FIG. 8 is an exemplary plurality of noise suppression gain curves.
FIG. 9 is an exemplary flow chart to describe the noise suppression method.
FIG. 10 is an exemplary ultrasound imaging system with automatic Doppler gain control and noise *suppression.*
FIG. 11A is an exemplary gain function processor *g*(*p*) and a g(p) generator.
FIG. 11B is an exemplary gain function processor *g*(*p*) with generator.
FIG. 12 is an exemplary Doppler spectrum with time.

### BEST MODE FOR CARRYING OUT THE INVENTION

Embodiments of the invention will be described with reference to the accompanying drawing figures wherein like numbers represent like elements throughout. Before embodiments of the invention are explained in detail, it is to be understood that the invention is not limited in its application to the details of the examples set forth in the following description or illustrated in the figures. The invention is capable of other embodiments and of being practiced or carried out in a variety of applications and in various ways. Also, it is to be understood that the phraseology and terminology used herein is for the purpose of description and should not be regarded as limiting. The use of "including," "comprising," or "having," and variations thereof herein is meant to encompass the items listed thereafter and equivalents thereof as well as additional items. The terms "mounted," "connected," and "coupled," are used broadly and encompass both direct and indirect mounting, connecting, and coupling. Further, "connected," and "coupled" are not restricted to physical or mechanical connections or couplings.

It should be noted that the invention is not limited to any particular software language described or that is implied in the figures. One of ordinary skill in the art will understand that a variety of alternative software languages may be used for implementation of the invention. It should also be understood that some of the components and items are illustrated and described as if they were hardware elements, as is common practice within the art. However, one of ordinary skill in the art, and based on a reading of this detailed description, would understand that, in at least one embodiment, components in the method and system may be implemented in software or hardware.

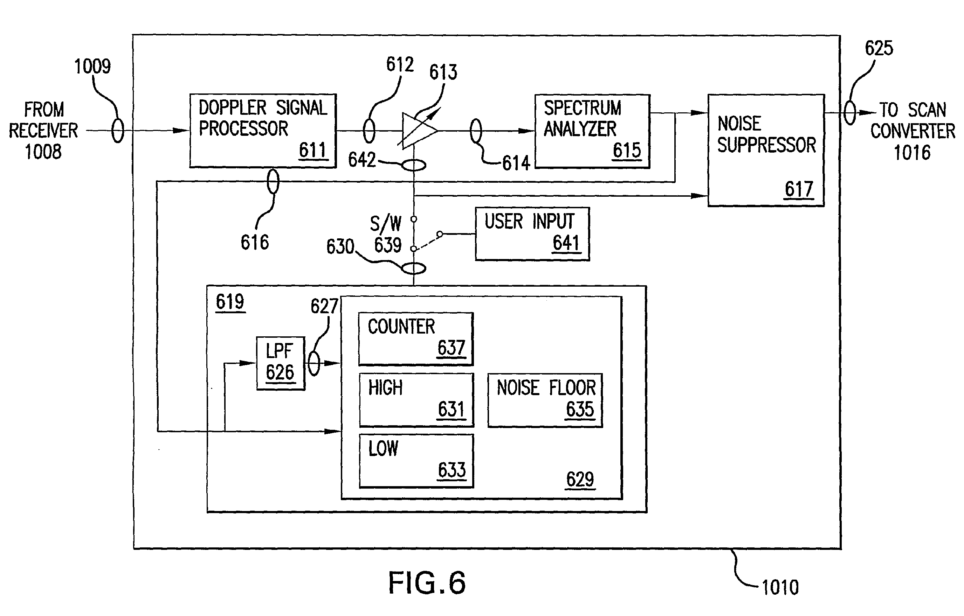

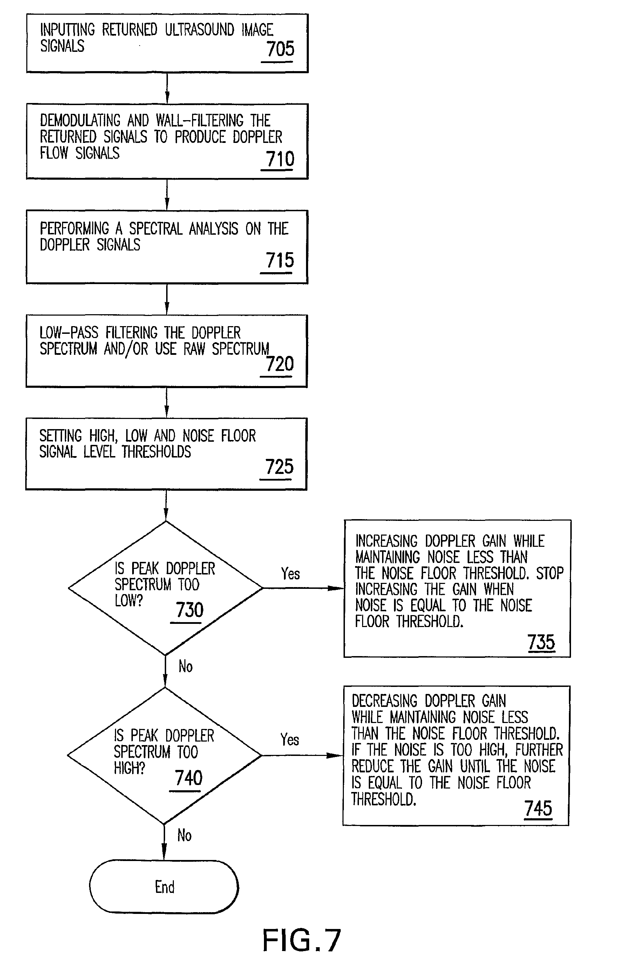

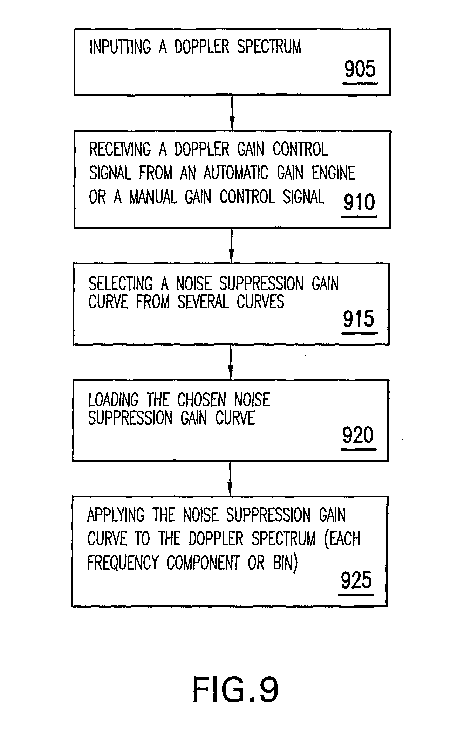

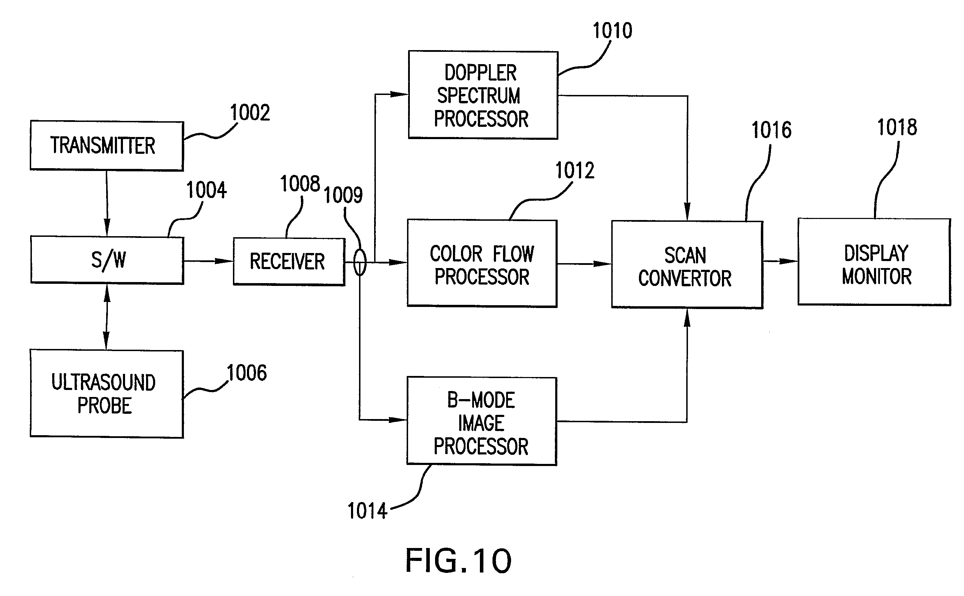

FIG. 10 shows an ultrasound system including a Doppler spectrum processor 1010 with the automatic Doppler gain and noise suppression system. FIG. 6 shows the Doppler processor 1010 with the automatic gain engine 619 and noise suppressor 617. FIG. 7 shows a flow chart to describe the automatic Doppler gain method. FIG. 9 shows a flow chart to describe the noise suppression method. An ultrasound signal is transmitted from an ultrasound probe 1006 driven by a transmitter 1002 through a transmit/receive switch 1004. A receiver 1008 receives the ultrasound signal from the probe 1006 through the switch 1004 and processes the signal 1009 (step 705).

The receiver 1008 outputs the processed signal 1009 to the Doppler spectrum processor 1010, a color flow processor 1012 and a B-mode image processor 1014. The Doppler spectrum processor 1010 processes the signal 1009 and outputs a Doppler spectrum to a scan converter 1016. The color flow processor 1012 processes the signal 1009 and outputs a color flow image to the scan converter 1016. The B-mode image processor 1014 processes the signal 1009 and outputs a B-mode image to the scan converter 1016. The scan converter 1016 receives one or more signals from the B-mode image, the color flow image and the Doppler spectrum and converts the images to a scan-converted image for output to a display monitor 1018.

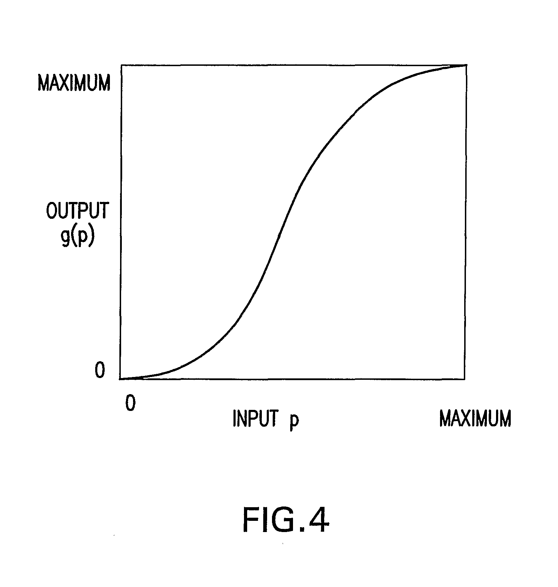

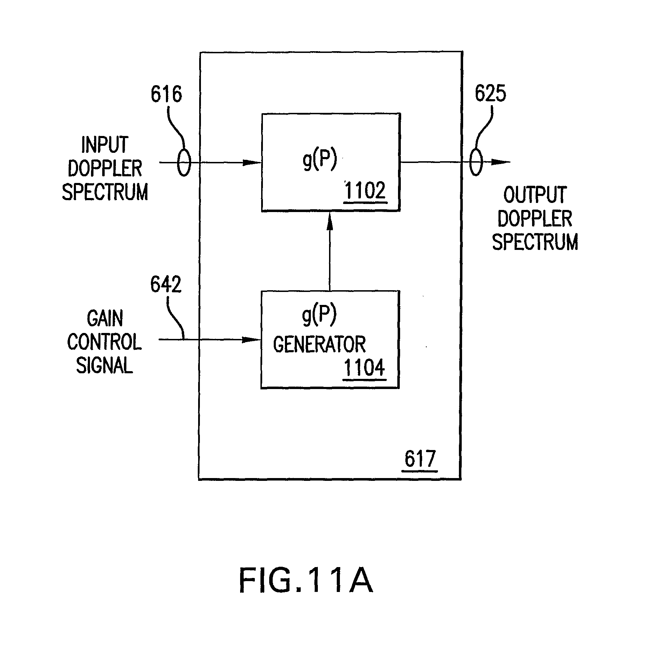

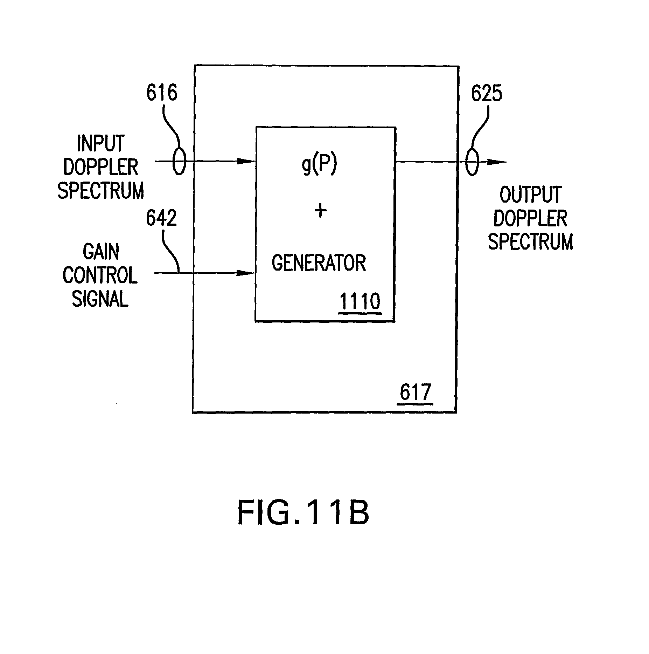

The processed signal 1009 is coupled to a Doppler signal processor 611 for computing Doppler flow signals 612 in the time domain (step 710). The Doppler flow signals 612 are coupled to a variable gain amplifier (VGA) 613 for adjusting the gain of the Doppler signals. The gain adjusted Doppler signals 614 are coupled to a spectrum analyzer 615 that converts the time domain Doppler signals into their spectrum frequency components (step 715). The frequency components, or spectrum 616, are coupled to the noise suppressor 617 and the automatic gain engine 619. The noise suppressor 617 has an input-output relationship which may be a curve *g*(*p*) as shown in FIG. 4. The noise suppressor 617 may be implemented as a look-up table (LUT) with the input-output relationship *g*(*p*) 1102 or 1110, or a calculator 1110 or a combination, and a gain curve generator 1104 which may also be a LUT or a calculator as shown in FIGs. 11A and 11B. For the case of a LUT combined with a calculator as the generator 1104, a noise suppression curve may be stored in the LUT, and the calculator receives the suppression curve and generates a curve corresponding to the gain control signal 642.

For the case of a LUT alone for the generator 1104, a plurality of noise suppression curves are stored in the LUT and a noise suppression curve is selected corresponding to the gain control signal 642. Alternately, a calculator alone as the generator 1104 can generate a noise suppression curve corresponding to the Doppler gain curve. The generator 1104 then transfers the curve to the gain function processor 1102 which may be a LUT and applies the noise suppression curve *g*(*p*) to the Doppler spectrum 616. Alternately, the gain function *g*(*p*) processor 1102 and the noise suppression curve generator 1104 can be implemented as one device 1110 as shown in FIG. 11B. A LUT with a Doppler spectrum 616 input and a gain control signal 642 input may be used. Alternately, the calculator 1110 may be used to generate a noise suppression curve as well as applying the gain function *g*(*p*) to the Doppler spectrum 616.

The noise suppressor 617 suppresses noise manifest on the Doppler spectrum 616. The noise suppressor 617 outputs a noise suppressed Doppler spectrum (output 625). The automatic gain engine 619 includes a low-pass filter 626 and a signal threshold processor 629. The low-pass filter 626 filters the spectrum frequency components 616 output by the spectrum analyzer 615, producing a smoothed spectrum 627, and outputs to the signal threshold processor 629. The raw Doppler spectrum 616 is also coupled to the signal threshold processor 629 (step 720).

The signal threshold processor 629 includes high 631, low 633 and noise floor 635 level thresholds for detecting the levels of the smoothed spectrum 627 and a frequency bin counter 637 for detecting frequency components. Likewise, the signal threshold processor 629 includes high 631, low 633 and noise floor 635 level thresholds for detecting the levels of the raw Doppler spectrum 616 and a frequency bin counter 637 for detecting frequency components (step 725). FIG. 3 shows an exemplary smoothed Doppler spectrum with the high 631, low 633 and noise floor 635 level thresholds against a maximum spectrum amplitude level. The maximum spectrum amplitude level is typically 255 (8-bit), 511 (9-bit), 1023 (10-bit), or other levels. The high signal level threshold 631 may be, for example, 255, 250, 225 or 200 for a maximum of 255. The low signal level threshold 633 may be, for example, 128, for the maximum spectrum level of 255, and the noise floor level threshold 635 may be, for example, 16 for the maximum spectrum level of 255.

The automatic gain engine 619 optimizes the Doppler flow signal gain by comparing the peak Doppler spectrum output 616 by the spectrum analyzer 615 to the high 631 and low 633 signal level thresholds. The frequency bin counter 637 counts a number of consecutive Doppler spectrum frequencies 616 whose amplitudes are greater than the high signal level threshold 631. The frequency bin counter 637 also counts a number of consecutive Doppler spectrum frequencies whose amplitudes are greater than the low signal level threshold 633. The frequency bin counter 637 also detects the maximum level of noise floor 301 which is a flat part in the Doppler spectrum.

FIG. 1 shows a Doppler spectrum 101 exhibiting a clipped 103 peak Doppler spectrum 627. Clipping occurs when the Doppler spectrum amplitude exceeds the maximum spectrum level. Clipping indicates that the Doppler gain is too high. In this invention, the Doppler gain 613 is considered too high if a number of consecutive spectrum frequencies (or frequency bins), whose amplitudes are greater than the high signal level threshold 631, is greater than a predetermined number, for example, 10.

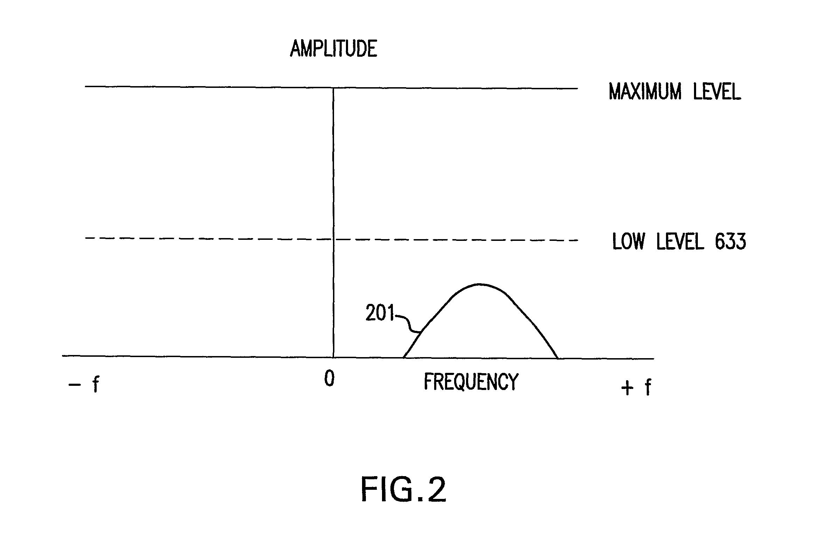

FIG. 2 shows a Doppler spectrum exhibiting a low 201 peak Doppler spectrum 627 or 616 amplitude (or power) which indicates a Doppler gain that is too low. In this invention, the gain (Doppler gain) of the variable gain amplifier 613 is considered too low if a number of consecutive spectrum frequencies (or frequency bins), whose amplitudes are greater than the low signal level threshold 633, is less than a predetermined number, for example, 10.

Instead of a raw (*i.e*, single) Doppler spectrum 616, a smoothed (low-pass filtered) Doppler spectrum 627 may be used with a smaller preset (count) number and/or a lower high signal level.

The automatic gain engine 619 detects a noise floor which may be spread across the entire frequency range since most electronic noise is random. When the Doppler spectrum is calculated, noise spreads over the entire frequency range due to its wideband nature. Noise is easily detected if the blood flow velocity is smaller than the maximum velocity or the Doppler spectrum bandwidth is smaller than the PRF. FIG. 3 shows a maximum noise floor 301 in conjunction with a Doppler spectrum and a deadband 303 between the high signal level 631 and low signal level 633 thresholds. A frequency band which consists of only the noise floor can be easily recognized as shown in FIG. 3 (low level ripple) and the maximum level 301 of the noise floor is determined in this frequency range. For example, an average amplitude of a predetermined number, for example, 10, of consecutive frequency components (bins) may be calculated for all spectrum frequency components excluding near the baseline (0 frequency) because the noise is absent in this area due to the wall filter's effects. The average amplitude from the noise floor region will be much smaller than that of the spectrum frequency components for blood flow as can be seen in FIG. 3. Thus, the noise floor area is determined in comparison to the blood flow area. The minimum average amplitude is obtained and is multiplied by a predetermined factor to estimate the maximum noise floor. Blood flow velocity changes with time as the blood velocity is high during systole and is low during diastole. Therefore, during diastole, the noise floor usually appears in high frequency region because the blood flow is low and high frequencies are absent (i.e. showing only noise floor). This can be further used to identify the noise floor.

If the peak Doppler spectrum 627 or 616 is less than the low signal level threshold 633, the automatic gain engine 619 generates a gain control signal 630 which is output to the variable gain amplifier 613 (step 730). The gain control signal 630 is coupled to the variable gain amplifier 613 through an automatic/manual Doppler gain mode switch 639. The switch 639 allows a user to select between the automatic gain control and the user gain control by switching between derived gain control signal 630 and a user adjusted manual gain control signal 641. The gain control signal 630 may be derived from several control strategies and corresponds to an amount of correction necessary to elevate the peak Doppler spectrum until a correct gain is achieved, i.e. the number of consecutive spectrum frequencies 627 whose amplitudes exceed the high level threshold 631, equals the predetermined number or the predetermined number minus a small preset number. If a noise floor 301 is present and rises commensurately above the noise floor level threshold 635 with the peak Doppler spectrum 627, the gain control signal 630 is adjusted, reducing the Doppler gain such that the noise floor is equal to or less than the noise floor level threshold 635 (step 735).

If the number of consecutive Doppler spectrum frequencies (i.e. frequency bins) whose amplitudes exceed the high level threshold 631 is more than the predetermined number, a high gain is detected and the automatic gain engine 619 generates a gain control signal 630 which is output to the variable gain amplifier 613 (step 740). The gain control signal 630 corresponds to an amount of correction necessary to decrease the peak Doppler spectrum 627 until a correct gain is achieved, i.e. the number of consecutive spectrum frequencies 627 or 616, whose amplitudes exceed the high level threshold 631, equals the predetermined number or the predetermined number minus a preset number. If a noise floor 301 is present and is greater than the noise floor level threshold 635, the gain control signal 630 is adjusted, reducing the Doppler gain such that the noise floor is equal to or less than the noise floor level threshold 635 (step 745).

If the peak Doppler spectrum 627 or 616 is less than or equal to the high signal level threshold 631 condition (i.e. if the number of consecutive spectrum frequencies, whose amplitudes exceed the high level, exceeds the predetermined number), and if a maximum noise floor 301 is greater than the noise floor level threshold 635, the gain control signal 630 is adjusted. The Doppler gain is reduced such that the maximum noise floor is equal to or less than the noise floor level threshold 635.

The noise suppressor 617 suppresses noise manifest on the Doppler signal 616. FIG. 9 shows a flow chart which describes the noise suppression method. The noise suppressor 617 is dependent on the gain control signal 642 since the noise floor varies with gain (Doppler gain) (steps 905, 910). If the Doppler gain is increased, the noise suppressor 617 receives the gain control signal 642 and selects a noise suppression gain curve from a plurality of gain curves stored or generated in the gain curve generator 1104 or 1110 (step 915).

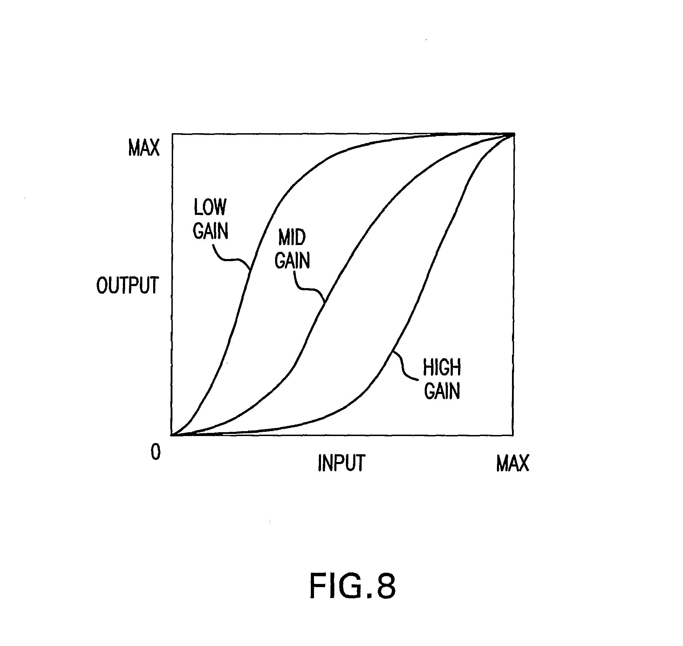

FIG. 8 shows an example of three noise suppression gain curves for low gain, mid gain, and high gain conditions stored or generated in the generator 1104 or 1110. The suppression gain curves stored or generated in the gain curve generator 1104 or 1110 correspond with a gain setting. If the Doppler gain is low as indicated by the gain control signal, the "low gain" noise suppression curve is selected or generated as shown in FIG.8. If the Doppler gain is middle, the "mid gain" noise suppression curve is selected or generated. If the gain is high, the "high gain" noise suppression curve is selected or generated. The selected noise suppression gain curve is loaded as the gain function *g*(*p*) in the gain function processor 1102 or 1110 (step 920). In another example, if the Doppler gain control signal 642 is set at 1, the #1 suppression curve is selected or generated. If the Doppler gain control signal 642 is set at 2, the #2 suppression curve is selected or generated. Likewise, if the Doppler gain control signal is *N*, the *N^{th}* suppression curve is selected or generated. The selected noise suppression gain curve is loaded as the gain function *g*(*p*) 1102 or 1110 (step 920). The noise suppressor 617 may comprise a calculator alone, a calculator with a LUT, or a plurality of LUTs, and uses the gain control signal 642 as shown in FIGs. 11A and 11B.

The noise suppressor 617 receives the Doppler spectrum 616 and converts each spectrum magnitude *p* using the response *g*(*p*) 1102 or 1110. The gain function *g*(*p*) 1102 or 1110 is the gain curve from the gain curve generator 1104 or 1110. FIG. 4 shows a gain function *g*(*p*) that is a curve.

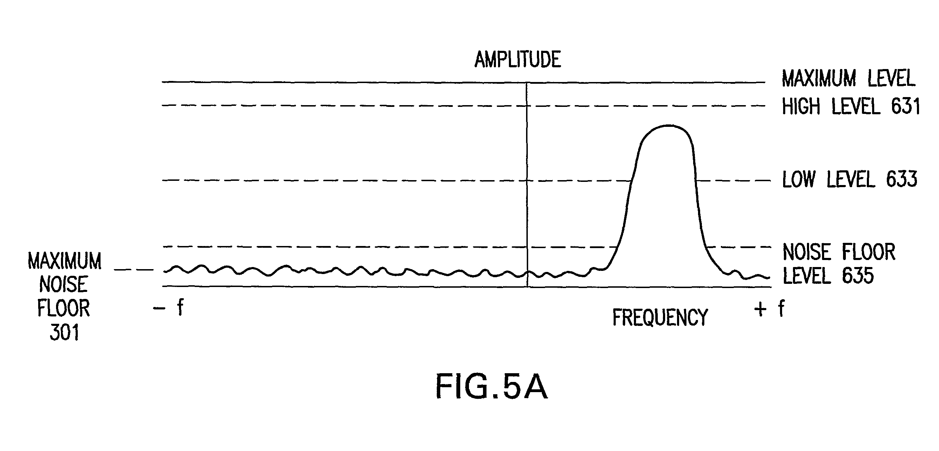

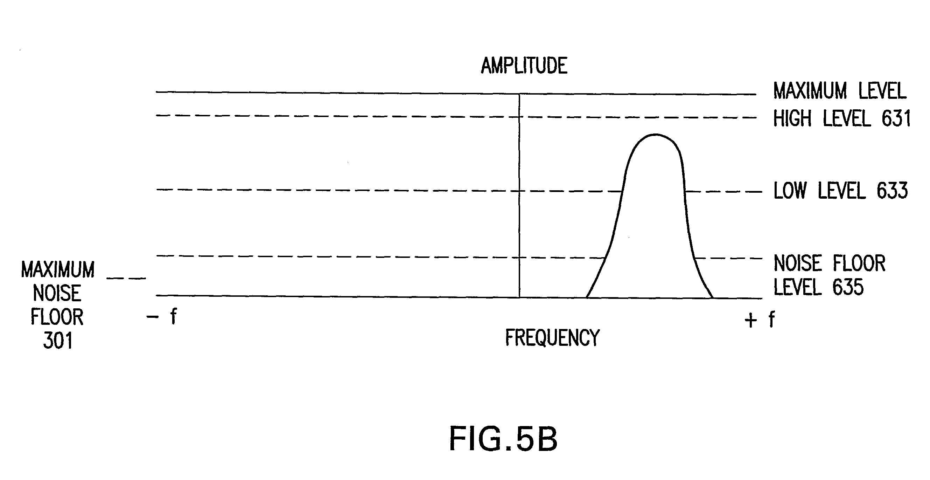

FIG. 5A shows a Doppler spectrum with noise. FIG. 5B shows the result of the noise suppressor 617 (step 925). The noise suppressor 617 applies a noise suppression curve technique, which lowers the noise floor.

## Claims

1. A method for automatically controlling the gain from a Doppler signal processor during ultrasound imaging comprising:
inputting returned ultrasound signals (705);
demodulating the returned ultrasound signals (710);
wall-filtering the returned ultrasound signals producing Doppler flow signals (710);
performing spectral analysis on the Doppler flow signals (715) producing a Doppler spectrum;
setting a high level signal threshold (631);
setting a low level signal threshold (633);
setting a noise floor level threshold (635);
detecting a peak Doppler spectrum level and a Doppler spectrum maximum noise floor from the Doppler flow signals;
increasing Doppler flow signal gain if the peak Doppler spectrum amplitude is less than the low level signal threshold (633) until the peak Doppler spectrum amplitude equals the high level signal threshold (631) (735) whereby if a noise floor (301) is present and was raised commensurately above the noise floor level threshold (635) with the peak Doppler spectrum amplitude, the Doppler flow gain signal is adjusted, reducing the Doppler flow gain such that the noise floor (301) is equal to or less than the noise floor level threshold (635); and
decreasing the Doppler flow signal gain if the peak Doppler spectrum amplitude is greater than the high level signal threshold (631) until the peak Doppler spectrum amplitude equals the high level signal threshold (631) (745) whereby if a noise floor (301) is present and is greater than the noise floor level threshold (635), the Doppler flow signal gain is adjusted, reducing the Doppler flow gain such that the noise floor (301) is equal to or less than the noise floor level threshold (635).

2. The method according to claim 1 further comprising smoothing the Doppler spectrum using a low-pass filter.

3. The method according to claim 1 wherein the determination of whether the peak Doppler spectrum amplitude is greater than the high level signal threshold (631) further comprises:
counting a number of consecutive Doppler spectrum frequency components whose amplitudes are greater than the high level signal threshold (631); and
comparing the number of consecutive Doppler spectrum frequency components whose amplitudes are greater than the high level signal threshold (631) with a predetermined number, wherein if the number of consecutive frequency components is greater than the predetermined number, the peak Doppler spectrum amplitude is greater than the high level signal threshold (631).

4. The method according to claim 1 wherein the determination of whether the peak Doppler spectrum is less than the low level signal threshold (633) further comprises:
counting a number of consecutive Doppler spectrum frequency components whose amplitudes are greater than the low level signal threshold (633); and
comparing the number of consecutive Doppler spectrum frequency components whose amplitudes are greater than the low level signal threshold (633) with a predetermined number, wherein if the number of consecutive frequency components is less than the predetermined number, the peak Doppler spectrum amplitude is less than the low level signal threshold (633).

5. The method according to claim 1 wherein detecting the Doppler spectrum maximum noise floor further comprises:
calculating an average amplitude of a predetermined number of the consecutive Doppler spectrum frequency components, for all spectrum frequency components excluding frequency components near a zero frequency baseline;
determining a minimum average amplitude among the average amplitudes; and
determining the maximum noise floor as the minimum average amplitude multiplied by a predetermined factor.

6. A system for automatically controlling the gain of a Doppler spectrum processor during ultrasound imaging comprising:
a receiver (1008) configured to receive returned ultrasound signals and having an output;
a Doppler signal processor (611) having an input coupled to the receiver (1008) output and an output, the Doppler signal processor (611) configured to demodulate and wall-filter the returned ultrasound signals and output Doppler flow signals;
a variable gain amplifier (613) having an input coupled to the Doppler signal processor (611) output, a gain control signal input and an output, the variable gain amplifier (613) configured to vary the gain of the Doppler flow signals;
a spectrum analyzer (615) having an input coupled to the variable gain amplifier (613) output and an output, the spectrum analyzer (615) configured to convert the Doppler flow signals into their corresponding frequency spectrum;
an automatic gain engine coupled to the spectrum analyzer (615) output, the automatic gain engine configured to receive the Doppler spectrum and detect a peak Doppler spectrum amplitude and a maximum noise floor wherein a gain control signal is calculated and coupled to the variable gain amplifier (615) gain control signal input based on the maximum noise floor present in the Doppler flow signals spectrum and predetermined high (631), low (633) and noise floor signal level (635) thresholds wherein if the peak Doppler spectrum amplitude is less than the low level signal threshold (633), the automatic gain engine is further configured to increase the Doppler gain signal until the peak Doppler spectrum equals the high level signal threshold (631) whereby if a noise floor (301) is present and was raised commensurately above the noise floor level threshold (635) with the peak Doppler spectrum amplitude, the Doppler flow gain signal is adjusted, reducing the Doppler flow gain such that the noise floor (301) is equal to or less than the noise floor level threshold (635); and wherein if the peak Doppler spectrum amplitude is greater than the high level signal threshold (631), the automatic gain engine is further configured to decrease the Doppler gain signal until the peak Doppler spectrum amplitude equals the high level signal threshold (631) whereby if a noise floor (301) is present and is greater than the noise floor level threshold (635), the Doppler flow signal gain is adjusted, reducing the Doppler flow gain such that the noise floor (301) is equal to or less than the noise floor level threshold (635).

7. The system according to claim 6 wherein the automatic gain engine further comprises a low-pass filter configured to smooth the Doppler spectrum.

8. The system according to claim 6 wherein the automatic gain engine is further configured to count a number of consecutive frequency components of the peak Doppler spectrum whose amplitudes are greater than the high level signal threshold (631) and compare the number of consecutive frequency components whose amplitudes are greater than the high level signal threshold (631) with a predetermined number, wherein if the number of consecutive frequency components is greater than the predetermined number, the peak Doppler spectrum amplitude is greater than the high level signal threshold (631).

9. The system according to claim 6 wherein the automatic gain engine is further configured to count a number of consecutive frequency components of the peak Doppler spectrum whose amplitudes are greater than the low level signal threshold (633) and compare the number of consecutive frequency components whose amplitudes are greater than the low level signal threshold (633) with a predetermined number, wherein if the number of consecutive frequency components is less than the predetermined number, the peak Doppler spectrum amplitude is less than the low level signal threshold (633).

10. The system according to claim 6 wherein the automatic gain engine is further configured to detect the Doppler spectrum maximum noise floor from an average amplitude of a predetermined number of the consecutive Doppler spectrum frequency components, for all spectrum frequency components excluding frequency components near a zero frequency baseline, and among the average amplitudes determines a minimum average amplitude wherein the maximum noise floor is the minimum average amplitude multiplied by a predetermined factor.

## Patentansprüche

1. Verfahren zum automatischen Steuern der Verstärkung von einem Doppler-Signalprozessor während einer Ultraschallbildgebung mit:
Einspeisen von zurückkommenden Ultraschallsignalen (705);
Demodulieren der zurückkommenden Ultraschallsignale (710);
Sperrfiltern der zurückkommenden Ultraschallsignale, wobei Doppler-Flusssignale (710) erzeugt werden;
Durchführen einer Spektralanalyse an den Doppler-Flusssignalen (715), wobei ein Doppler-Spektrum erzeugt wird;
Setzen eines Signalschwellenwerts mit einem hohen Niveau (631);
Setzen eines Signalschwellenwerts mit einem niedrigen Niveau (633);
Setzen eines Schwellenwerts eines Grundrauschniveaus (635);
Feststellen eines Niveaus einer Spitze des Doppler-Spektrums und eines maximalen Grundrauschens des Doppler-Spektrums von den Doppler-Flusssignalen;
Erhöhen der Verstärkung des Doppler-Flusssignals, wenn die Amplitude der Spitze des Doppler-Spektrums kleiner ist als der Signalschwellenwert mit dem niedrigen Niveau (633), bis die Amplitude der Spitze des Doppler-Spektrums dem Signalschwellenwert mit dem hohen Niveau (631) gleicht (735), wobei, wenn ein Grundrauschen (301) vorhanden ist und entsprechend über den Schwellenwert des Grundrauschniveaus (635) mit der Amplitude der Spitze des Doppler-Spektrums erhöht wurde, die Verstärkung des Doppler-Flusssignals angepasst wird, wobei die Verstärkung des Doppler-Flusses reduziert wird, so dass das Grundrauschen (301) gleich oder geringer ist als der Schwellenwert des Grundrauschniveaus (635); und
Verringern der Verstärkung des Doppler-Flusssignals, wenn die Amplitude der Spitze des Doppler-Spektrums größer ist als der Signalschwellenwert mit dem hohen Niveau (631), bis die Amplitude der Spitze des Doppler-Spektrums dem Signalschwellenwert mit dem hohen Niveau (631) gleicht (745), wobei, wenn ein Grundrauschen (301) vorhanden ist und größer ist als der Schwellenwert des Grundrauschniveaus (635), die Verstärkung des Doppler-Flusssignals angepasst wird, wobei die Verstärkung des Doppler-Flusses reduziert wird, so dass das Grundrauschen (301) gleich oder geringer ist als der Schwellenwert des Grundrauschniveaus (635).

2. Verfahren nach Anspruch 1,
welches ferner das Glätten des Doppler-Spektrums unter Verwendung eines Tiefpassfilters aufweist.

3. Verfahren nach Anspruch 1,
wobei das Bestimmen ob die Amplitude der Spitze des Doppler-Spektrums gröβer ist als der Signalschwellenwert mit dem hohen Niveau (631) ferner aufweist:
Zählen einer Anzahl von aufeinanderfolgenden Frequenzkomponenten des Doppler-Spektrums, deren Amplituden größer sind als der Signalschwellenwert mit dem hohen Niveau (631); und
Vergleichen der Anzahl von aufeinanderfolgenden Frequenzkomponenten des Doppler-Spektrums, deren Amplituden größer sind als der Signalschwellenwert mit dem hohen Niveau (631) mit einer vorgegebenen Anzahl,
wobei, wenn die Anzahl von aufeinanderfolgenden Frequenzkomponenten größer ist als die vorgegebene Anzahl, die Amplitude der Spitze des Doppler-Spektrums größer ist als der Signalschwellenwert mit dem hohen Niveau (631).

4. Verfahren nach Anspruch 1,
wobei das Bestimmen, ob die Spitze des Doppler-Spektrums kleiner ist als der Signalschwellenwert mit dem niedrigen Niveau (633) ferner aufweist:
Zählen einer Anzahl von aufeinanderfolgenden Frequenzkomponenten des Doppler-Spektrums, deren Amplituden größer sind als der Signalschwellenwert mit dem niedrigen Niveau (633); und
Vergleichen der Anzahl von aufeinanderfolgenden Frequenzkomponenten des Doppler-Spektrums, deren Amplituden größer sind als der Signalschwellenwert mit dem niedrigen Niveau (633), mit einer vorgegebenen Anzahl, wobei, wenn die Anzahl von aufeinanderfolgenden Frequenzkomponenten geringer ist als die vorgegebene Anzahl, die Amplitude der Spitze des Doppler-Spektrums kleiner ist als der Signalschwellenwert mit dem niedrigen Niveau (633).

5. Verfahren nach Anspruch 1,
wobei das Feststellen des maximalen Grundrauschens des Doppler-Spektrums ferner aufweist:
Berechnen einer mittleren Amplitude von einer vorgegebenen Anzahl von den aufeinanderfolgenden Frequenzkomponenten des Doppler-Spektrums, für alle Frequenzkomponenten des Spektrums ausschließend Frequenzkomponenten nahe einer Null-Frequenzgrundlinie;
Bestimmen einer minimalen mittleren Amplitude unter den mittleren Amplituden; und
Bestimmen des maximalen Grundrauschens als die minimale mittlere Amplitude multipliziert mit einem vorgegebenen Faktor.

6. System zum automatischen Steuern der Verstärkung eines Doppler-Spektrumsprozessors während einer Ultraschallbildgebung mit:
einem Empfänger (1008), welcher ausgebildet ist, zurückkommenden Ultraschallsignale zu empfangen und welcher einen Ausgang hat;
einem Doppler-Signalprozessor (611), welcher einen an den Ausgang des Empfängers (1008) gekoppelten Eingang und einen Ausgang hat, wobei der Doppler-Signalprozessor (611) ausgebildet ist, die zurückkommenden Ultraschallsignale zu demodulieren und sperrzufiltern und Doppler-Flusssignale auszugeben;
einem Verstärker mit variabler Verstärkung (613), welcher einen an den Ausgang des Doppler-Signalprozessors (611) gekoppelten Eingang, einen Verstärkungssteuerungssignaleingang und einen Ausgang hat, wobei der Verstärker mit variabler Verstärkung (613) ausgebildet ist, die Verstärkung der Doppler-Flusssignale zu variieren;
einem Spektralanalysator (615), welcher einen an den Ausgang des Verstärkers mit variabler Verstärkung (613) gekoppelten Eingang und einen Ausgang hat, wobei der Spektralanalysator (615) ausgebildet ist, die Doppler-Flusssignale in ihre zugehörigen Frequenzspektren zu konvertieren;
einer automatischen Verstärkungs-Engine, welche an den Ausgang des Spektralanalysators (615) gekoppelt ist, wobei die automatische Verstärkungs-Engine ausgebildet ist, das Doppler-Spektrum zu empfangen und eine Amplitude der Spitze des Doppler-Spektrums und ein maximales Grundrauschen zu bestimmen, wobei ein Verstärkungssteuerungssignal berechnet wird und an den Verstärkungssteuerungssignaleingang des Verstärkers mit variabler Verstärkung (615) gekoppelt ist, basierend auf dem maximalen Grundrauschen, welches in dem Doppler-Flusssignalspektrum vorhanden ist, und vorgegebenen Signalschwellenwerten mit hohen Niveau (631), niedrigen Niveau (633) und Grundrauschniveau (635) basiert;
wobei, wenn die Amplitude der Spitze des Doppler-Spektrums kleiner ist als der Signalschwellenwert mit dem niedrigen Niveau (633), die automatische Verstärkungs-Engine ferner ausgebildet ist, die Verstärkung des Doppler-Signals zu erhöhen, bis die Spitze des Doppler-Spektrums dem Signalschwellenwert mit dem hohen Niveau (631) gleicht, wobei, wenn ein Grundrauschen (301) vorhanden ist und entsprechend über den Schwellenwert des Grundrauschniveaus (635) mit der Amplitude der Spitze des Doppler-Spektrums erhöht wurde, die Verstärkung des Doppler-Flusssignals angepasst wird, wobei die Verstärkung des Doppler-Flusses reduziert wird, so dass das Grundrauschen (301) gleich oder geringer ist als der Schwellenwert des Grundrauschniveaus (635); und
wobei, wenn die Amplitude der Spitze des Doppler-Spektrums größer ist als der Signalschwellenwert mit dem hohen Niveau (631), die automatische Verstärkungs-Engine ferner ausgebildet ist, die Verstärkung des Doppler-Signals zu verringern bis die Amplitude der Spitze des Doppler-Spektrums dem Signalschwellenwert mit dem hohen Niveau (631) gleicht, wobei, wenn ein Grundrauschen (301) vorhanden ist und größer ist als der Schwellenwert des Grundrauschniveaus (635), die Verstärkung des Doppler-Flusssignals angepasst wird, wobei die Verstärkung des Doppler-Flusses reduziert wird, so dass das Grundrauschen (301) gleich oder geringer ist als der Schwellenwert des Grundrauschniveaus (635).

7. System nach Anspruch 6,
wobei die automatische Verstärkungs-Engine ferner einen Tiefpassfilter aufweist, welcher ausgebildet ist, das Doppler-Spektrum zu glätten.

8. System nach Anspruch 6,
wobei die automatische Verstärkungs-Engine ferner ausgebildet ist, eine Anzahl von aufeinanderfolgenden Frequenzkomponenten der Spitze des Doppler-Spektrums zu zählen, deren Amplituden größer sind als der Signalschwellenwert mit dem hohen Niveau (631) und die Anzahl von aufeinanderfolgenden Frequenzkomponenten, deren Amplituden größer sind als der Signalschwellenwert mit dem hohen Niveau (631), mit einer vorgegebenen Anzahl zu vergleichen, wobei, wenn die Anzahl von aufeinanderfolgenden Frequenzkomponenten größer ist als die vorgegebene Anzahl, die Amplitude der Spitze des Doppler-Spektrums größer als der Signalschwellenwert mit dem hohen Niveau (631) ist.

9. System nach Anspruch 6,
wobei die automatische Verstärkungs-Engine ferner ausgebildet ist, eine Anzahl von aufeinanderfolgenden Frequenzkomponenten der Spitze des Doppler-Spektrums zu zählen, deren Amplituden größer sind als der Signalschwellenwert mit dem niedrigen Niveau (633) und die Anzahl von aufeinanderfolgenden Frequenzkomponenten, deren Amplituden größer sind als der Signalschwellenwert mit dem niedrigen Niveau (633), mit einer vorgegebenen Anzahl zu vergleichen, wobei, wenn die Anzahl von aufeinanderfolgenden Frequenzkomponenten geringer ist als die vorgegebene Anzahl, die Amplitude der Spitze des Doppler-Spektrums kleiner als der Signalschwellenwert mit dem niedrigen Niveau (633) ist.

10. System nach Anspruch 6,
wobei die automatische Verstärkungs-Engine ferner ausgebildet ist, das maximale Grundrauschen des Doppler-Spektrums von einer mittleren Amplitude einer vorgegebenen Anzahl der aufeinanderfolgenden Frequenzkomponenten des Doppler-Spektrums festzustellen, für alle Frequenzkomponenten des Spektrums ausschließend Frequenzkomponenten nahe einer Null-Frequenzgrundlinie, und unter den mittleren Amplituden eine minimale mittlere Amplitude bestimmt, wobei das maximale Grundrauschen die minimale mittlere Amplitude multipliziert mit einem vorgegebenen Faktor ist.

## Revendications

1. Méthode pour contrôler automatiquement le gain d'un processeur de signal Doppler pendant une imagerie à ultrasons comprenant :
une entrée de signaux à ultrasons de retour (705),
une démodulation des signaux à ultrasons de retour (710),
un filtrage via un filtre coupe-bande des signaux à ultrasons de retour produisant les signaux Doppler de débit (710),
un accomplissement d'une analyse spectrale sur les signaux Doppler de débit (715) produisant un spectre Doppler,
une mise d'une valeur seuil de signal de niveau élevé (631),
une mise d'une valeur seuil de signal de niveau faible (633),
une mise d'une valeur seuil de niveau bruit de fond (635),
une détection d'un niveau de spectre Doppler maximal et d'un bruit de fond maximal du spectre Doppler à partir des signaux Doppler de débit,
une augmentation du gain de signal Doppler de débit si l'amplitude du spectre Doppler maximale est inférieure à la valeur seuil de signal de niveau faible (633) jusqu'à ce que l'amplitude du spectre Doppler maximale soit égale à la valeur seuil de signal de niveau élevé (631) (735) tandis que si un bruit de fond (301) est présent et a été élevé proportionnellement au-dessus de la valeur seuil de niveau bruit de fond (635) avec l'amplitude du spectre Doppler maximale, le signal Doppler de débite de gain est ajusté, réduisant le gain de débit Doppler de sorte que le bruit de fond (301) soit équivalent à ou inférieur à la valeur seuil de niveau bruit de fond (635), et
une diminution du gain de signal Doppler de débit si l'amplitude du spectre Doppler maximale est plus grande que la valeur seuil de signal de niveau élevé (631) jusqu'à ce que l'amplitude du spectre Doppler maximale soit égale à la valeur seuil de signal de niveau élevé (631) (745) tandis que si un bruit de fond (301) est présent et est plus grand que la valeur seuil de niveau bruit de fond (635), le gain de signal Doppler de débit est ajusté, réduisant le gain de débit Doppler de sorte que le bruit de fond (301) soit équivalent à ou inférieur à la valeur seuil de niveau bruit de fond (635).

2. Méthode selon la revendication 1,
comprenant en outre l'aplatissement du spectre Doppler en utilisant un filtre passe-bas.

3. Méthode selon la revendication 1,
la détermination de la question de savoir si l'amplitude du spectre Doppler maximale est plus grande que la valeur seuil de signal de niveau élevé (631) comprenant en outre:
un comptage d'un nombre de composants de fréquence de spectre Doppler consécutifs dont les amplitudes sont plus grandes que la valeur seuil de signal de niveau élevé (631), et
une comparaison du nombre de composants de fréquence de spectre Doppler consécutifs dont les amplitudes sont plus grandes que la valeur seuil de signal de niveau élevé (631) avec un nombre prédéterminé,
tandis que si le nombre de composants de fréquence consécutifs est supérieur au nombre prédéterminé, l'amplitude du spectre Doppler maximale est supérieure à la valeur seuil de signal de niveau élevé (631).

4. Méthode selon la revendication 1,
la détermination de la question de savoir si l'amplitude du spectre Doppler maximale est inférieure à la valeur seuil de signal de niveau faible (633) comprenant en outre:
un comptage d'un nombre de composants de fréquence de spectre Doppler consécutifs dont les amplitudes sont plus grandes que la valeur seuil de signal de niveau faible (633), et
une comparaison du nombre de composants de fréquence de spectre Doppler consécutifs dont les amplitudes sont plus grandes que la valeur seuil de signal de niveau faible (633) avec un nombre prédéterminé,
tandis que si le nombre de composants de fréquence consécutifs est inférieur au nombre prédéterminé, l'amplitude du spectre Doppler maximale est inférieure à la valeur seuil de signal de niveau faible (633).

5. Méthode selon la revendication 1,
la détection du bruit de fond maximal du spectre Doppler comprenant en outre:
un calcul d'une amplitude moyenne d'un nombre prédéterminé de composants de fréquence de spectre Doppler consécutifs, pour tous les composants de fréquence spectre à l'exclusion des composants de fréquence proches d'une base zéro fréquence,
une détermination d'une amplitude moyenne minimale parmi les amplitudes moyennes, et
une détermination d'un bruit de fond maximal en tant que l'amplitude moyenne minimale multipliée par un facteur prédéterminé.

6. Système pour contrôler automatiquement le gain d'un processeur de spectre Doppler pendant une imagerie à ultrasons comprenant :
un receveur (1008) configuré pour recevoir des signaux à ultrasons de retour et pour avoir une sortie,
un processeur de signal Doppler (611) ayant une entrée couplée à la sortie du receveur (1008) et une sortie, le processeur de signal Doppler (611) configuré pour démoduler et filtrer via un filtre coupe-bande les signaux à ultrasons de retour et pour sortir des signaux Doppler de débit,
un amplificateur de gain variable (613) ayant une entrée couplée à la sortie du processeur de signal Doppler (611), une entrée de signal de contrôle de gain et une sortie, l'amplificateur de gain variable (613) configuré pour varier le gain de signal Doppler de débit,
un analyseur de spectre (615) ayant une entrée couplée à la sortie de l'amplificateur de gain variable (613) et une sortie, l'analyseur de spectre (615) configuré pour convertir les signaux Doppler de débit dans leur spectre de fréquence correspondant,
un moteur de gain automatique couplé à la sortie de l'analyseur de spectre (615), le moteur de gain automatique configuré pour recevoir le spectre Doppler et détecter une amplitude de spectre Doppler maximale et un bruit de fond maximal tandis qu'un signal de contrôle de gain est calculé et couplé à une entrée de signal de contrôle de gain d'un amplificateur de gain variable (615) basé sur le bruit de fond maximal présent dans le spectre de signal Doppler de débit et des valeurs seuil de signal de niveau élevé (631), de niveau faible (633) et de niveau bruit de fond (635) prédéterminées,
tandis que si l'amplitude du spectre Doppler maximale est inférieure à la valeur seuil de signal de niveau faible (633), le moteur de gain automatique est en outre configuré pour augmenter le signal Doppler de gain jusqu'à ce que le spectre Doppler maximal soit équivalent à la valeur seuil de signal de niveau élevé (631)
tandis que si un bruit de fond (301) est présent et a été élevé proportionnellement au-dessus de la valeur seuil de niveau bruit de fond (635) avec l'amplitude du spectre Doppler maximale, le signal Doppler de gain de débit est ajusté, réduisant le gain de débit Doppler de sorte que le bruit de fond (301) soit équivalent ou inférieur à la valeur seuil de niveau bruit de fond (635), et
tandis que si l'amplitude du spectre Doppler maximale est plus grande que la valeur seuil de signal de niveau élevé (631), le moteur de gain automatique est en outre configuré pour réduire le signal Doppler de gain jusqu'à ce que l'amplitude du spectre Doppler maximale soit équivalente à la valeur seuil de signal de niveau élevé (631) tandis que si un bruit de fond (301) est présent et est plus grand que la valeur seuil de niveau bruit de fond (635), le gain de signal Doppler de débit est ajusté, réduisant le gain de débit Doppler de sorte que le bruit de fond (301) soit équivalent ou inférieur à la valeur seuil de niveau bruit de fond (635).

7. Système selon la revendication 6,
le moteur de gain automatique comprenant en outre un filtre passe-bas configuré pour aplatir le spectre Doppler.

8. Système selon la revendication 6,
le moteur de gain automatique étant également configuré pour compter un nombre de composants de fréquence consécutifs du spectre Doppler maximal dont les amplitudes sont plus grandes que la valeur seuil de signal de niveau élevé (631) et comparer le nombre de composants de fréquence consécutifs dont les amplitudes sont plus grandes que la valeur seuil de signal de niveau élevé (631) avec un nombre prédéterminé, tandis que si le nombre de composants de fréquence consécutifs est plus grand que le nombre prédéterminé, l'amplitude du spectre Doppler maximale est plus grande que la valeur seuil de signal de niveau élevé (631).

9. Système selon la revendication 6,
le moteur de gain automatique étant en outre configuré pour compter un nombre de composants de fréquence consécutifs du spectre Doppler maximal dont les amplitudes sont plus grandes que la valeur seuil de signal de niveau faible (633) et comparer le nombre de composants de fréquence consécutifs dont les amplitudes sont plus grandes que la valeur seuil de signal de niveau faible (633) avec un nombre prédéterminé, tandis que si le nombre de composants de fréquence consécutifs est inférieur au nombre prédéterminé, l'amplitude du spectre Doppler maximale est inférieure à la valeur seuil de signal de niveau faible (633).

10. Système selon la revendication 6,
le moteur de gain automatique étant en outre configuré pour détecter le bruit de fond du spectre Doppler à partir d'une amplitude moyenne d'un nombre prédéterminé de composants de fréquence consécutifs d'un spectre Doppler, pour tous les composants de fréquence de spectre, à l'exclusion des composants de fréquence proches d'une base zéro fréquence, et déterminant, parmi les amplitudes moyennes, une amplitude moyenne minimale, le bruit de fond maximal étant l'amplitude moyenne minimale multipliée par un facteur prédéterminé.
